# EUROPEAN PATENT SPECIFICATION

(11) **EP 0 629 293 B1**
(45) Date of publication and mention of the grant of the patent: **26.07.2000**
(21) Application number: 93905501.8
(22) Date of filing: 04.03.1993
(51) Int. Cl.: G01N 33/564, G01N 33/68, G01N 33/58, G01N 33/543, A61K 38/45, C12N 5/08, C12N 9/02, C12N 15/81

(54) **ASSAY FOR ADRENAL AUTOANTIBODIES**
NACHWEISVERFAHREN FÜR ADRENALE AUTOANTIKÖRPER
ESSAI DE DEPISTAGE D'AUTO-ANTICORPS SURRENAL

(30) Priority: 07.03.1992 GB 9205040; 31.07.1992 GB 9216306
(43) Date of publication of application: 21.12.1994
(73) Proprietor: RSR LIMITED, Pentwyn, Cardiff CF2 7HE (GB)
(72) Inventor: FURMANIAK-WEHR, Jadwiga Maria, Lakeside, Cardiff CF2 6DR (GB)
(74) Representative: Bizley, Richard Edward
(86) International application number: GB9300443
(87) International publication number: WO9318406

(56) References cited:
- EP-A- 0 340 878
- FEBS LETTERS vol. 231, no. 1, April 1988, AMSTERDAM NL pages 25 - 28 J. FURMANIAK; D. TALBOT; D. REINWEIN; G. BENKER; F. M. CREAGH AND B. REES SMITH 'Immunoprecipitation of human adrenal microsomal antigen' cited in the application
- THE LANCET vol. 339, 28 March 1992, LONDON GB pages 770 - 773 KAI KROHN ET AL. 'Identification by molecular cloning of an autoantigen associated with Addison's didease as steroid 17alpha-hydroxylase' cited in the application
- MOLECULAR AND CELLULAR ENDOCRINOLOGY vol. 68, no. 1, 2 January 1990, SHANNON, IE pages 29 - 34 M. L. CHOUINARD AND H. R. FEVOLD 'ACTH-induced increases in rabbit adrenal immunoreactive P-450-17alpha and P-450-21' cited in the application
- THE JOURNAL OF CLINICAL ENDOCRINOLOGY & METABOLISM vol. 70, no. 1, January 1990, PHILADELPHIA, PA, US pages 95 - 99 GEORGE M. BRIGHT AND INDERJIT SINGH 'Adrenal Autoantibodies Bind to Adrenal Subcellular Fractons Enriched in Cytochrome-c Reductase and 5'-Nucleotidase' cited in the application
- PROCEEDINGS OF THE NATIONAL ACADEMY OF SCIENCES OF USA vol. 83, no. 14, July 1986, WASHINGTON US pages 5111 - 5115 PERRIN C. WHITE, MARIA I. NEW, AND BO DUPONT 'Structure of human steroid 21-hydroxylase genes' cited in the application
- Bumpus, J.A. and K.M. Dus (1982) J. Biol. Chem. 257:12696-12704

## Description

The present invention relates to adrenal autoantigen. More particularly, it relates to immunoassays for adrenal autoantibodies involving adrenal autoantigen. The invention further relates to methods of using adrenal autoantigen in the diagnosis of autoimmune Addison's disease.

Addison's disease is a disorder characterised by failure of the adrenal gland and is often an autoimmune disorder involving destruction of the adrenal cortex and the presence of adrenal autoantibodies in the patient's serum. The adrenal cortex is responsible for producing several steroid hormones including cortisol, aldosterone and testosterone. In autoimmune Addison's disease and other forms of the disease, levels of these hormones are reduced. This reduction in hormone levels is responsible for the clinical symptoms of the disease which include low blood pressure, muscle weakness, increased skin pigmentation and electrolyte imbalance.

Autoantibodies to the adrenal cortex were first described in 1957 using the technique of complement fixation¹. Subsequently other laboratories confirmed the presence of these autoantibodies using complement fixation or immunofluorescence²⁻⁴. Radioimmunoassay and ELISA techniques have also been described using crude adrenal membrane preparations⁵⁻⁶.

The preparation of purified rabbit adrenal autoantigen was reported in 1973⁷ but the isolation of human adrenal autoantigen remained an elusive goal. The human adrenal autoantigen(s) has/have been localised to the microsomal fraction of adrenal tissue homogenates and in 1988 it was shown that the antigen is a membrane protein with a molecular weight of about 55,000¹¹. In 1990 there was described an assay to detect binding of adrenal autoantibodies to subcellular fractions of the human adrenal gland⁸. The assay indicated that the autoantibody has a plasma membrane and/or microsomal source and was described as providing a more rational approach to "eventual" antigen isolation and purification.

Measurement of adrenal autoantibodies is important in the diagnosis of Addison's disease and currently immunofluorescence is used routinely.

The present inventor has for the first time succeeded in isolating and characterising an adrenal antigen. The isolation and characterisation of the antigen enables the production of the antigen, for example, by recombinant techniques, the generation of antibodies to the antigen, purification of adrenal autoantibody and assays for the antigen. Such assays are useful for the diagnosis of latent or actual autoimmune Addison's disease.

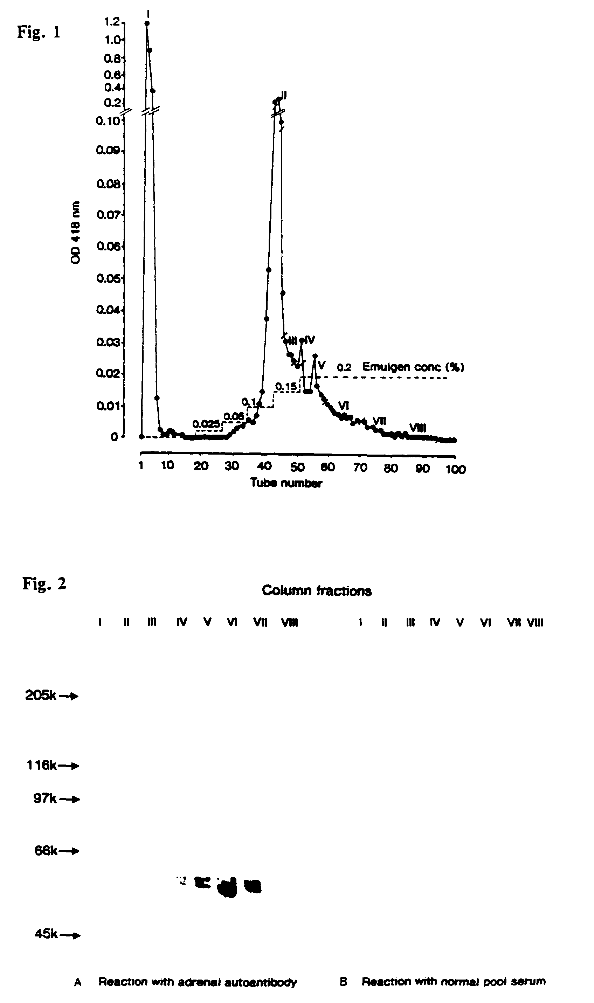

The present invention provides a method for detecting adrenal autoantibody in a sample, comprising contacting with the sample:
(a) a protein comprising an epitope for adrenal autoantibody, having an observed molecular weight of from about 50,000 to about 60,000 and obtainable by:
   homogenising mammalian adrenal glands,
   subjecting the homogenate to differential centrifugation to obtain a microsome fraction,
   suspending the microsome fraction in a phosphate buffer,
   centrifuging the suspension in the presence of sodium cholate to form a supernatant,
   adding polyethylene glycol and further sodium cholate to the supernatant and mixing the supernatant,
   centrifuging the thus mixed supernatant to form a pellet,
   resuspending the pellet in aqueous sodium cholate to form a suspension,
   dialysing the suspension against aqueous sodium cholate to form a solubilised microsome preparation, and
   purifying the solubilised microsome preparation by chromatography on an Octyl-Sepharose™ CL-4B column equilibrated with 50 mM phosphate buffer, pH 7.0, 20% glycerol, 0.1 mM EDTA, 0.1 mM DTT, and eluted with 0.2% Emulgen™ to obtain a column fraction corresponding to peak VI of Figure 1;
(b) the adrenal protein (a) modified by one or more amino acid modifications and comprising an epitope for the adrenal autoantibody; or
(c) a fragment of modified or unmodified adrenal protein (a) comprising an epitope for the adrenal autoantibody.

The protein is preferably obtainable from human adrenal glands.

Experiments indicate that the protein obtainable by the above techniques is steroid 21-hydroxylase (p450c21). Steroid 21-hydroxylase is an enzyme prominent in the zona glomerulosa of the adrenal cortex and which catalyses one step of the biological pathway from cholesterol to hyrdrocortisone. cDNA encoding 21-hydroxylase has been cloned and sequenced and an amino acid sequence derived for the protein¹⁰.

The adrenal protein obtainable as described above may be modified by one or more amino acid modifications (deletions, additions or substitutions) which do not cause loss of adrenal autoantibody binding properties.

The invention includes the use of a fragment of the protein which comprises an epitope for adrenal autoantibody. Suitable fragments include synthetic peptides.

The protein may be in crude or purified form. Preferably, it is substantially pure. The protein may be native protein derived from a human or animal source or it may be of recombinant origin.

The proteins which characterise the invention may be in the form of a modified protein or a protein fragment as described above. The proteins (including modified proteins and fragments) may be labelled. The proteins are preferably human. The invention includes protein fragments as defined in (c) above and which are labelled. The invention further provides a synthetic peptide comprising an epitope to adrenal autoantibody and obtainable from a protein as defined in (a) above.

In yet other aspects the invention resides in kits for detecting adrenal autoantibodies in a sample. In one aspect the kit comprises the adrenal protein. The invention includes the use in an assay for adrenal autoantibodies of a protein or a protein fragment of the invention or of an antibody to such a protein or protein fragment.

The invention opens the way to the therapy of adrenal autoimmune disorders as described more fully hereafter, by the disruption of the lymphocytes involved in the disorder. In particular, the invention provides pharmaceutical formulations comprising a protein or protein fragment, especially a peptide, of the invention for pharmaceutical use. In another aspect there is provided a pharmaceutical formulation comprising a protein or protein fragment of the invention formulated for pharmaceutical use; the formulation may be a composition which comprises a pharmaceutically acceptable diluent, excipient or carrier as well as the active protein or protein fragment.
Figure 1 is an elution profile of proteins eluted from a column chromatograph;
Figure 2 shows a Western blot of eluted column fractions contacted with (A) serum containing adrenal autoantibodies and (B) normal serum;
Figure 3 shows the protein staining pattern of eluted column fractions after gel electrophoresis;
Figure 4 is a dot blot assay of eluted column fractions contacted with (A) serum containing adrenal autoantibodies and (B) normal serum;
Figure 5 shows a dot blot assay of eluted column fraction VI contacted with ten different serum samples.
Figure 6 is a Western blot analysis of eluted column fraction VI; and
Figure 7 is a Western blot analysis of the protein of column fraction VI after SDS-PAGE analysis and electroelution.

The invention relates to assays for adrenal autoantibodies. The assays involve the use of antigenic protein described in more detail below.

### The Antigenic Protein

The protein useful in the invention is a protein obtainable from the microsome fraction of human or animal adrenal glands. The protein is an antigen for adrenal autoantibodies. The protein is further characterised by an observed molecular weight of from about 50,000 to 60,000 and preferably from about 52,000 to 58,000 (eg approximately 55,000). The molecular weight is that observed against the following standards in analysis by gel electrophoresis using a 9% polyacrylamide gel : myosin (205,000), beta-galactosidase (116,000), phosphorylase b (97,000), bovine serum albumin (66,000), ovalbumin (45,000) and carbonic anhydrase (29,000).

The adrenal protein preferably binds to human adrenal autoantibodies but less preferably may bind exclusively to animal adrenal autoantibodies.

The protein may be native protein extracted from human adrenal glands or, less preferably, adrenal glands from another mammal. The protein may be obtained by the following illustrative procedure. Adrenal glands are homogenised and the homogenate subjected to differential centrifugation to obtain a microsome fraction. The microsome fraction is suspended, eg in phosphate buffer containing glycerol.

The suspension is then centrifuged, typically in the presence of sodium cholate and normally also EDTA and dithiothreitol (DTT). After centrifugation polyethlene glycol (PEG) and further sodium cholate and normally glycerol, EDTA and DTT are added to the supernatant and the preparation mixed before further centrifuging. The pellet resulting from centrifuging is resuspended in aqueous sodium cholate normally containing glycerol, EDTA and DTT and dialysed against aqueous sodium cholate, again normally containing glycerol, EDTA and DTT.

After dialysis, the solubilised microsomes are preferably centrifuged, to remove any precipitated material. The solubilised microsome preparation may be stored at -70°C.

Throughout the procedure, the liquids are buffered at pH7, for example using phosphate buffer.

The solubilised microsome preparation is further purified by chromatography, for example using Octyl-Sepharose™ CL-4B and, as the eluant, phosphate buffer (which may contain glycerol, EDTA and DTT), followed by increasing concentrations of one or more detergents, for example that sold under the trade mark EMULGEN.

Column fractions are typically monitored by optical absorbance.

The column fraction containing the adrenal autoantigen may be determined by Western blotting. The column fractions are separated by gel electrophoresis followed by blotting onto nitrocellulose. The nitrocellulose is reacted with serum from a patient with a high level of adrenal autoantibodies and incubated with for example anti-human immunoglobulin-horseradish peroxidase conjugate and, reacted with a chemiluminescent substance. Chemiluminescence is detected with photographic film.

The above procedures are described to illustrate the invention by way of example only, and alternative protocols may be followed to obtain crude, purified or substantially pure antigen.

As described in more detail in the Examples, the adrenal auroantigen isolated by the described procedures was found to have a molecular weight of 55,000 ± 5,000. The protein was found to have the biochemical characteristics of steroid 21-hydroxylase and to react on Western blots with rabbit antibodies to recombinant 21-hydroxylase. Absorption of the native human adrenal protein with human adrenal autoantibodies prevented the subsequent reaction of the native human protein with rabbit antibodies to 21-hydroxylase in Western blot analysis. In addition, human adrenal autoantibodies reacted with recombinant 21-hydroxylase expressed in yeast. These experiments indicate that the protein is 21-hydroxylase.

Whilst the adrenal protein is preferably of human origin, the invention embraces antigens derived from animals. The antigen may be obtained by purification of animal or, preferably, human adrenal microsome fractions or by expression of recombinant protein.

As the antigen there may also be used synthetic peptides containing an epitope against adrenal autoantibody. Synthetic peptides may be prepared by any known synthetic procedure, for example. Such synthetic peptides represent a fragment of the native or modified protein.

### Steroid 21-hydroxylase

As described elsewhere herein, the adrenal protein has immunological properties which equate it with steroid 21-hydroxylase.

Steroid 21-hydroxylase is an enzyme which has been studied because deficiency in 21-hydroxylase activity results in inefficient synthesis of cortisol and elevated levels of corticotropin. Such 21-hydroxylase deficiency is one of several syndromes termed congenital adrenal hyperplasia and is inherited as a monogenic autosomal recessive trait. A plasmid designated pC21a comprising a portion of the coding sequence of bovine 21-hydroxylase was prepared for use in the diagnosis of congenital adrenal hyperplasia⁹. Plasmid pC21a was subsequently used to prepare a plasmid pC21/3c (pCD//pC21/3c) containing an insert encoding the complete cDNA for 21-hydroxylase except for about 30 base pairs at the 5' end¹⁰.

Plasmid pC21/3c has been sequenced¹⁰ and deposited at the American Type Culture Collection (ATCC) under ATCC Nos 57420 (freeze dried E. Coli containing the plasmid and 57421 (purified plasmid DNA). Both deposits are freely available to the public.

Saccharomyces cerevisiae capable of producing steroid 21-hydroxylase may be made by a method comprising ligating into a yeast expression vector the steroid 21-hydroxylase gene sequence obtainable from plasmid pCD//pC21/3c (ATCC 57421) modified by replacement of the bases coding for the first 13 amino acids with the bases coding for a yeast leader sequence, and transforming saccharomyces cerevisiae with the resultant vector.

Preferably, the steroid 21-hydroxylase gene sequence obtainable from said plasmid is subcloned as a BamHI fragment into vector pTZ18, and the resultant vector containing a 21-hydroxylase gene sequence is digested to obtain a fragment comprising a coding region for 21-hydroxylase and ligated into yeast expression vector pYES, as cut with BamHI and SphI, using a BamHI - NarI linker comprising 11 base pairs of non-coding and 42 base pairs of coding STE2 gene sequence.

Included in the invention is a method of producing a yeast expression vector comprising a DNA sequence encoding steroid 21-hydroxylase, comprising ligating into a yeast expression vector the steroid 21-hydroxylase gene sequence obtainable from plasmid pCD//pC21/3c (ATCC 57421) modified by replacement of the bases coding for the first 13 amino acids with the bases coding for a yeast leader sequence and a method of expressing steroid 21-hydroxylase, comprising culturing Saccharomyces cerevisiae transformants prepared by a method of the invention.

Steroid 21-hydroxylase may therefore be used in assays and kits for detecting adrenal autoantibody, especially in the diagnosis of Addison's disease. Steroid 21-hydroxylase may also be used in the therapy of adrenal autoimmune disorders.

The expression of bovine 21-hydroxylase as part of a process for the preparation of steroids is described in EP 0340878 (Gist-Brocades).

The 21-hydroxylase is preferably human 21-hydroxylase , but may alternatively be an animal (e.g. bovine) 21-hydroxylase. The 21-hydroxylase may be purified from human or animal sources but is preferably recombinant 21-hydroxylase. The 21-hydroxylase may have a native sequence or may be modified by one or more amino acid alterations which do not result in loss of antigenicity towards adrenal autoantibodies.

Also useful in the invention are fragments of 21-hydroxylase which retain the desired antigenic property.

The protein or protein fragment may be in crude, purified or pure form.

### Cloning Antigenic Protein

Recombinant protein may be prepared by isolating polyadenylated adrenal mRNA and reverse transcribing it into cDNA to prepare a cDNA library. A host organism is transformed with the cDNA using an appropriate expression vector and the transformants screened for organisms expressing the adrenal autoantigen, which organisms may then be cloned. Hosts may be prokaryotic or eukaryotic, for example E. Coli, yeast or CHO cells. Of course, when a host is transformed with a vector, appropriate promoter-operator sequences are also introduced in order for the protein to be expressed. The protein obtained may be glycosylated or unglycosylated.

An illustrative procedure for preparing recombinant adrenal protein will now be described in more detail by way of example only. Polyadenylated adrenal RNA is isolated and the cDNA sequence based on the RNA sequence is generated with the use of reverse transcriptase. The cDNA is then cloned into a bacteriophage vector (eg lambda gt10, lambda gt11, lambda Zap) and an adrenal cDNA library is created. The library is then screened using 32-P labelled oligonucleotide probes synthesised on the basis of known parts of the amino acid sequence of adrenal autoantigen. The library (when subcloned into the expression vector, eg lambda gt11) can alternatively be screened with autoantibodies to adrenal antigen or with monoclonal antibodies. In this case the positive plaques are identified in a reaction with radiolabelled Protein A or enzyme-labelled anti-IgG.

Positive plaques (ie containing the cDNA sequence complementary to the probes or expressing the antibody binding protein) are purified to homogeneity and ligated to create a full coding sequence. The full-length cDNA is then sequenced by the Sanger method and subcloned into a plasmid vector, eg pBR322, pTZ18 or Bluescript™.

Alternatively, the gene coding for adrenal autoantigen can be synthesised in a polymerase chain reaction (PCR). In this method, the first strand of cDNA is synthesised from RNA using reverse transcriptase and then used as a template for DNA amplification with Taq DNA polymerase. The primers for initiation of each round of amplification are synthesised based on the known amino acid sequence of the N terminus of the adrenal protein. In this case a modification of cDNA amplification using one-sided (anchored) PCR is used. The amplified gene is purified on an agarose gel and cloned into plasmid vectors as above.

For expression of recombinant protein, the full length of the isolated gene is cloned into a mammalian expression vector (eg pRC/CMV). CHO cells, for example, are then transfected (using the CaPO₄ precipitation method) with vectors containing the adrenal autoantigen gene. Cells are collected, concentrated by centrifugation and a microsomal preparation prepared. The presence of recombinant proteins in the microsomal fraction is detected by Western blotting using adrenal autoantibodies and the material purified using Octyl Sepharose chromatography as described above in relation to purification of native adrenal autoantigen.

Glycosylation of the recombinant protein may be studied using lectins and compared to the native protein.

Detailed methodology of cloning techniques may be found in J Fambrook; E F Fritch and T Maniatis, Molecular Cloning - A Laboratory Manual. Cold Spring Harbor Laboratory, Cold Spring Harbor, New York, Second Edition 1989.

A specific method of expressing human steroid 21-hydroxylase will now be described.

Human 21-hydroxylase may be expressed using the 21-hydroxylase gene in plasmid pCD//pC21/3c¹⁰. The purified plasmid is publically available from ATCC deposit No. 57421. Alternatively, a suitable plasmid may be prepared following the procedure of White et al¹⁰.

In an exemplary technique, the 21-hydroxylase gene in pCD//pC21/3c was modified by replacing the first 13 amino acids with the first 14 amino acids of STE2 gene and placed under the control of GAL1 promoter in pYES 2.0 (Invitrogen).

In this technique the 21-hydroxylase gene sequence was subcloned from pCD//pC21/3c as a BamHI fragment into the vector pTZ18 (Pharmacia). Digestion with SphI (Northumbria Biologicals Ltd, Cramlington NE23 9HL, UK) followed by partial digestion with NarI (Northumbria Biologicals Ltd, Cramlington NE23 9HL, UK) yielded a large fragment comprising the entire coding region of 21-hydroxylase apart from 41 base pairs at the 5' end. This was then ligated into pYES cut with BamHI (Northumbria Biologicals Ltd. Cramlington NE23 9HL. UK) and SphI using a BamHI-NarI linker comprising 11 base pairs of non-coding and 42 base pairs of STE2 coding sequence⁵⁸. Transformants of Saccharomyces cerevisiae (C13 ABYS 8B - Yeast Genetic Stock Center, Berkeley, California) were grown in selective media and used to inoculate expression cultures in YEP-glucose (2%) or YEP-galactose (2%). [YND is yeast nitrogen base from Difco Laboratories + 2% dextrose and YEP is yeast extract + 2% peptone (both from Oxoid)]. After 48 hours at 30°C, the cells were harvested, broken by vortexing with glass beads in 1% sodium deoxycholate and analysed on SDS-PAGE followed by Western blotting.

Bovine 21-hydroxylase may be expressed as described in EP 0340878.

The protein may be a variant of the native form containing amino acid alterations which do not result in loss of antigenicity to adrenal autoantibodies.

Such modified proteins may be prepared, for example, by the use of oligonucleotide directed mutagenesis with a synthetic oligonucleotide (typically 7 to 24 bases long) that is complementary to the wild-type sequence but which contains one or more base changes compared to the wild-type sequence.

The size of the oligonucleotide primer is determined by the requirement for stable hybridization of the primer to the region of the gene in which the mutation is to be induced, and by the limitations of the available methods for synthesizing oligonucleotides. In general the overall length of the oligonucleotide will be such as to optimize stable, unique hybridization at the mutation site with the 5' and 3' extensions from the mutation site being of sufficient size to avoid editing of the mutation by the exonuclease activity of the DNA polymerase. Oligonucleotides used for mutagenesis usually contain from about 7 and more usually about 12 to about 24 bases, preferably from about 14 to about 20 bases and still more preferably from about 15 to about 18 bases. They will usually contain at least about three bases at the centre or positions immediately 3' of the centre of the altered or missing codon.

The primer is hybridized to single-stranded phage such as M13 into which a strand of DNA of the invention has been cloned. It will be appreciated that the phage may carry either the sense strand or antisense strand of the DNA. When the phase carries the antisense strand the primer is identical to the region of the sense strand that contains the codon(s) to be mutated except for a mismatch with said codon(s) that defines a deletion of the codon or a triplet that codes for another amino acid.

When the phage carries the sense strand the primer is complementary to the region of the sense strand that contains the codon(s) to be mutated except for an appropriate mismatch in the triplet that is paired with the or each codon to be mutated. The hybridization is carried out at a temperature which will usually range between about 0°C and 70°C, more usually about 10°C to 50°C.

Melting temperature (Tm) of synthesised oligonucleotides is calculated and used as a guidance to hybridisation temperature conditions. Usually the template and the oligonucleotides are heated briefly to 20°C above the estimated Tm. Hybrids form as the temperature of the reaction mixture drops below Tm. The resulting mixture of double-stranded heteroduplex DNA is then transfected directly into a bacterial host and mutants can be identified by screening plaques by hybridisation, using the radiolabelled mutagenic oligonucleotide as a probe. Hybridisation is usually carried out under conditions that allow the labelled oligonucleotide to form hybrids with both mutant and wild-type DNA. By progressively increasing the stringency of the subsequent washes it is possible to establish conditions under which hybrids between mutagenic oligonucleotide and wild-type DNA will dissociate and perfect hybrids formed by the oligonucleotide and desired mutant will not dissociate. Identified in this way mutants are then purified and the presence of the desired mutation confirmed by DNA sequencing of the mutagenised and adjacent regions or of the full insert sequence carried in the vector.

After the hybridization, the primer is extended on the phage DNA by reaction with DNA polymerase I, T₄ DNA polymerase, reverse transcriptase or other suitable DNA polymerase. The resulting dsDNA is convened to closed circular dsDNA by treatment with a DNA ligase such as T₄ DNA ligase. DNA molecules containing single-stranded regions may be destroyed by S1 endonuclease treatment.

The resulting mutational heteroduplex is then used to transform a competent host organism or cell. Replication of the heteroduplex by the host provides progeny from both strands. Following replication the mutant gene may be isolated from progeny of the mutant strand, inserted into an appropriate expression vector, and the vector used to transform a suitable host organism or cell. Exemplary vectors and hosts are described above.

Directed mutagenesis techniques are well known and are described in the literature²⁴⁻²⁸:

The invention accordingly includes not only DNA sequences encoding protein of the invention but also DNA sequences which hybridise to such sequences and encode an antigenic determinant to adrenal autoantibody. Such hybridisable sequences may be sequences encoding a modified protein or a protein fragment, whether of modified or unmodified protein. Hybridisation conditions are described, for example, by Smith M and Gillam S²⁵ and by Fambrook et al. (supra)⁵⁹. Modified DNA is normally capable of hybridising to DNA sequences encoding protein of the invention under conventional hybridising conditions and preferably under stringent hybridising conditions.

### Antibodies to the Antigenic Protein

In some assays, antibodies to the adrenal protein are useful. Such antibodies may be prepared by any known method, for example.

Polyclonal antibodies may be prepared by administering the adrenal autoantigen or cells expressing the antigen to an animal in order to induce the production of serum containing polyclonal antibodies.

Monoclonal antibodies can be prepared using the hybridoma technique of Kohler and Milstein²⁹. Further exemplary literature references are listed in the attached bibliography³⁰⁻³⁴. According to the hybridoma method, a human or other animal or lymphocytes therefrom is/are immunised with a protein or protein fragment of the invention. Lymphocytes from the immunised individual or culture are fused with myelomas into single cells. The resulting continuous cell lines are screened for antibody producing hybrids. Such hybrids may be cultured and the antibody collected.

The antibodies useful in the invention include not only intact molecules, including human monoclonal antibodies, but also fragments thereof which are capable of binding to the antigenic protein. Also useful are recombinant antibodies.

Antibodies to 21-hydroxylase may be made by such techniques as described above.

### Labelling

The assays of the invention normally involve antigen or antibody labelled so as to be detectable. Numerous methods of labelling are known and any such known technique may be used, for example. Examples of the extensive literature describing labelling techniques are to be found in the attached bibliography³⁵.

Exemplary labels are radioactive labels, enzymes, fluorescent compounds, chemiluminescent compounds, bioluminescent compounds and metal chelates. The binding of such labels to the antigen or antibody may be performed using any conventional technique, for example.

### Assays

The assays of the invention include those assays known to the skilled person for the quantitative or non-quantitative detection of antibodies and all involve contacting antigenic protein (the adrenal protein obtainable as previously described or fragments thereof) with a sample. The assays may be competitive or non-competitive and examples are radioimmunoassays (RIA), enzyme immunoassays (EIA), and enzyme-linked immunosorbent assays (ELISA). The reader is referred to the literature for details of suitable assays³⁵.

The competitive assays of the invention include assays which involve contacting an antigenic protein (which expression embraces protein fragments) with a biological sample (typically serum or urine). Normally the sample is diluted, for example, 1 part sample in from 10 to 30 parts diluent such as phosphate buffered saline or another buffer. Either simultaneously or subsequently there is added labelled antibody against the antigenic protein. The labelled antibody and any antibody in the sample compete to bind to the antigen and the bound labelled antibody after competition is measured. The antigenic protein in such techniques is preferably immobilised on a substrate by known techniques such as covalent bonding via an amide or ester linkage or adsorption, for example.

The invention also includes sandwich assays (eg RIA, EIA, ELISA) in which the antigenic protein immobilised on a substrate is contacted with a biological sample suspected of containing adrenal autoantibodies. After washing, there is added labelled antigenic protein or labelled anti-adrenal autoantibody antibody, either of which selectively binds to any adrenal autoantibody which has bound to the immobilised antigen. The appropriate detection technique for the label is then employed, eg quantitative or non-quantitative detection of radioactive emissions or colorimetric techniques. Sandwich assays can also be employed using, as the labelled ligand for the antibody, any agent capable of recognising an antibody, for example labelled anti-immunoglobulins, protein A or protein G.

Other assays comprise contacting a sample with labelled antigenic protein. separating bound and unbound labelled antigen (eg using a column having adsorbed thereon antibody to the antigen) and detecting the labelling by a quantitative or non-quantitative technique.

Assays may also be performed by contacting with a sample immobilised anti-antigenic protein antibody having bound thereto labelled antigen and detecting labelled antigen bound to immobilised antibody or labelled antigen bound to antibody in the sample.

### Two illustrative assay techniques for adrenal autoantibodies

### 1. Assay based on a radioactive label

Purified adrenal autoantigen is labelled with a radioactive label such as ¹²⁵I using one of many well-known techniques. The labelled material will then be incubated (1h at room temperature) with a suitably diluted (eg 1 in 20 in phosphate buffered saline) serum sample. Adrenal autoantibodies present in the test sample will bind to the ¹²⁵I-labelled adrenal autoantigen and the resulting complex is precipitated by addition of antibodies to human immunoglobulins or addition of a similar reagent (eg solid phase Protein A). The amount of ¹²⁵I-labelled antigen in the precipitate is then determined. The amount of adrenal autoantibody in the test serum sample will be a function of the amount of radioactivity precipitated. The amount of adrenal autoantibody can be expressed as the amount of radioactivity in the pellet or more usually by including dilution of an adrenal autoantibody positive reference serum in the assay.

### 2. Assay based on an enzyme label

Purified adrenal autoantigen is coated onto plastic wells of ELISA plates either directly onto plain wells or indirectly. The indirect method could involve coating the wells first with a monoclonal or polyclonal antibody to adrenal autoantigen (the antibody would be selected so as not to bind to the same site as adrenal autoantibodies) followed by addition of adrenal autoantigen. Several other indirect coating methods are well known.

After coating with autoantigen, suitably diluted (eg 1 in 20 in phosphate buffered saline) test sera are added to the wells and incubated (1h at room temperature) to allow binding of adrenal autoantibody to the antigen coated onto the wells.

The wells are then washed and a reagent such as anti-human IgG conjugated to horseradish peroxidase is added. After further incubation (eg 1h at room temperature) and washing, an enzyme substrate such as orthophenylene diamine is added and the colour generated measured by light absorbance. The amount of adrenal autoantibody in the test sample will be a function of the final colour intensity generated. Results can be expressed as light absorbance or more usually by including dilution of an adrenal autoantibody positive reference serum in the assay.

The assays of the invention involving the interaction of antigenic protein and a sample may be performed using a kit comprising antigenic protein. The sandwich assays of the invention may conveniently be performed using a kit comprising an antigenic protein, normally immobilised on a substrate, and labelled anti-adrenal autoantibody antibody or labelled antigenic protein.

The invention also includes kits for use in competitive assays comprising antigenic protein and labelled antibody to the antigenic protein. The antigenic protein is preferably immobilised on a substrate.

Further included in the invention are kits comprising labelled antigenic protein for use in simple assays involving detection of antigen/antibody complex. Other kits of the invention comprise anti-antigenic protein antibody which is immobilised on a substrate and which has bound thereto labelled antigenic protein.

In those kits comprising immobilised protein, the protein is preferably immobilised on the internal surface of a plastics tube.

The invention includes assays and kits as described above which use 21-hydroxylase as the antigen.

In a variant of the invention, the antigenic protein is replaced by antibody to the autoantibody. This variant is not suitable for sandwich assays using ligands which bind to any antibody.

The invention also includes the use of antibody against the adrenal protein of the invention to detect steroid 21-hydroxylase. Methods and kits as described above may be used for such assays. The antibody may be human monoclonal antibody but alternatively is human polyclonal antibody or animal polyclonal or monoclonal antibody. Optionally, the antibody used in assays of the invention may comprise more than one antibody.

### Therapy of Adrenal Autoimmune Disease

It is thought that autoimmune diseases are caused at least in part by autoantigens persistently activating T cells³⁶. The autoantigen in the case of adrenal autoimmune disease can be any epitope of the antigenic molecule which is recognised by a T cell receptor capable of helping a B cell make antibody to the antigenic molecule, or a T cell otherwise involved in the autoimmune disorder.

Adrenal autoimmune disorders should therefore be treatable by disrupting the action of T cells involved in the disorder. T cells such as, for example, those infiltrating the adrenal gland, in lymphoid organs or in the circulation can act as T helper (Th) cells in responding to adrenal autoantigen epitopes. Such Th cells help B cells make specific anti-autoantigen antibodies. T cells can also act in mediating cell-mediated immune responses and act on adrenal cortex cells by the release of cytokines or directly. The destruction of adrenal cortex cells can result, when cytotoxic T cells specific for the adrenal autoantigen are activated, or by an inflammatory response mediated by a different T cell class.

If such T cells were interfered with, the production of anti-autoantigen antibodies and of T cells destructive of adrenal cortex cells would be suppressed. The T cells could be disrupted by a protein or protein fragment (eg peptide) capable of binding to the T cell receptor (TCR) of an adrenal autoantigen specific T cell, ie containing a T cell epitope of the autoantigen.

Such a protein or peptide acts as a competitive antagonist for the autoantigen in the adrenal cortex and thereby inhibits antigen specific cellular interactions which result in the production of adrenal autoantibody or adrenal autoantigen specific cell-mediated immunity. Exemplary references describing the use of peptides to treat autoimmune disease are included in the attached bibliography³⁶⁻⁴².

Another therapeutic approach is to suppress the autoimmune response to adrenal autoantigen by administering adrenal autoantigen specific T cells4³⁻⁴⁹ or a protein or protein fragment (eg peptide) mimicking the TCR of such cells⁴⁸⁻⁴⁹. Administration of such a "vaccine" induces "counter-autoimmunity" which is thought to be mediated by T cells specific to the TCR of the autoimmune T cells^{43,48,50,51}.

The autoimmune response may also be suppressed by T suppressor (Ts) cells capable of specifically recognising an anti-adrenal autoantigen B cell or T cell^{52,53}.

Such therapy may be effected by administering to the patient a protein or protein fragment comprising an adrenal autoantigen epitope capable of inducing Ts cells or by administering adrenal autoantigen - specific Ts cells capable of suppressing an anti-adrenal autoantigen response.

The T cell epitope or epitopes of adrenal autoantigen may be determined by, for example, standard techniques in the art.

The autoimmune TCR specific to the adrenal autoantigen may be characterised using methods described in the literature⁵⁴. Disruption of interaction of the autoimmune T cells with the adrenal autoantigen may alleviate or prevent adrenal autoimmune disease. T cells which will disrupt such interaction and which are specific for the autoimmune TCR for adrenal autoantigen may be prepared⁵⁵.

The present invention provides pharmaceutical (including veterinary pharmaceutical) formulations comprising the adrenal protein obtainable from human or animal adrenal glands as defined above, which protein may optionally be modified by one or more amino acid alterations. The invention also includes formulations comprising a fragment of such protein, eg a peptide containing an antigenic determinant to adrenal autoantibody.

In another aspect, the invention provides pharmaceutical formulations comprising 21-hydroxylase or a fragment thereof, eg a peptide containing an antigenic determinant to adrenal autoantibody.

The invention also includes pharmaceutical formulations comprising a ligand, eg a peptide, capable of binding to the TCR of an adrenal autoantigen specific T cell or B cell.

In other aspects, the invention provides T cells specific for adrenal autoantigen and proteins or protein fragments (eg peptides) which mimick the receptor of such T cells. Included in the invention are counter-autoimmune T cells, ie T cells induced by such adrenal autoantibody specific T cell or TCR mimicking protein or protein fragment. Pharmaceutical compositions including a pharmaceutically acceptable diluent, excipient or carrier as well as any of the aforesaid agents are further provided by the invention.

Also provided by the invention are Ts cells capable of interacting specifically with an anti-adrenal autoantigen B cell or T cell and pharmaceutical formulations comprising such cells. Similarly included is the use of the adrenal protein defined above or of an 21-hydroxylase or a fragment of either (eg a peptide comprising a suitable epitope) to generate such anti-autoimmune T cells.

### Other Uses of the Antigenic Proteins

Antigenic protein may be used to purify adrenal autoantibody, for example by adsorbing the adrenal autoantigen or 21-hydroxylase on a substrate, passing a sample containing the antibody over the substrate and then washing the substrate to release the antibody.

The adrenal autoantibody may be used to raise antibody to the adrenal autoantibody for use in assays or, for example, for screening transformed host cells to detect expression of recombinant adrenal autoantibody, which itself could be labelled and used in a competitive assay, for example.

### Example 1

### Preparation and solubilisation of adrenal microsomes.

Human adrenal glands were recovered from the redundant tissue surrounding kidneys removed from cadaveric donors and then stored at -70°C in small (100mg) pieces. A microsome fraction was isolated from adrenal tissue homogenates by differential centrifugation¹¹⁻¹³ and suspended in 100mM phosphate buffer pH 7.0 containing 20% glycerol (1 ml of buffer for each g of starting adrenal tissue). The protein concentration was then estimated¹⁴ and the mixture adjusted to a final concentration of 25-30mg of adrenal protein per ml.

Sodium cholate (solid) was then added to a final concentration of 3mg of cholate per 1mg of adrenal proteins and the mixture was adjusted to a final concentration of 0.1mM EDTA and 0.1mM dithiothreitol (DTT). Sodium cholate was allowed to dissolve by mixing on a magnetic stirrer at 4°C for 1h and then the mixture was centrifuged at 100,000 x g for 1h at 4°C. After centrifugation, an equal volume of 50% PEG 6000 in 100mM phosphate buffer pH 7.0, 20% glycerol, 0.1mM DTT, 0.1mM EDTA was added to the supernatant and the preparation mixed on a magnetic stirrer for 30min at 4°C. This was followed by centrifugation at 12,000 x g for 1h at 4°C. The pellet was then resuspended in 3% sodium cholate in 100mM phosphate buffer pH 7.0 containing 20% glycerol, 0.1mM EDTA, 0.1mM DTT (1.3ml per 1g equivalent of starting adrenal tissue) and dialysed overnight at 4°C against 0.3% sodium cholate in 50mM phosphate buffer pH7.0, 20% glycerol, 0.1mM EDTA, 0.1mM DTT (basal buffer). After dialysis the solubilised microsomes were spun at 12,000 x g for 15 min at 4°C to remove any precipitated material.

The protein concentration of the solubilised microsome preparation was then estimated¹⁴ and the preparation stored in aliquots at -70°C.

### Example 2

### Purification of adrenal autoantigen.

The methods described by Kominami et al¹⁵ and Bumpus and Dus¹⁶ were used. In particular, Octyl-Sepharose™ CL-4B (Pharmacia LKB) was extensively washed in 50mM phosphate buffer pH 7.0, 20% glycerol, 0.1mM EDTA, 0.1mM DTT according to the manufacturer's instructions, poured into 1 x 10cm column and equilibrated with basal buffer.

6ml aliquots of solublished microsomes were applied to the column at 25ml/h and eluted initially with basal buffer. 3.3ml fractions were collected and monitored by light absorbance at 280nm and 418nm. Washing of the column was continued until the OD 280nm was below 0.01 and OD 418nm was zero. Further elution from the column was then carried out using increasing concentrations of Emulgen™ 913 (Kayo-Atlas, Tokyo) in the basal buffer (step-wise increases; 0.025%, 0.05%, 0.1%, 0.15% and 0.2%; 25ml each step).

The column fractions were monitored at 418nm (monitoring at 280nm is not possible in the presence of Emulgen™) and aliquots (0.5ml) taken from each fraction. The elution profile of proteins from the column is shown in Figure 1. Aliquots from groups of fractions were then pooled, concentrated (Centricon™ 10 microconcentrators) and analysed by gel electrophoresis. In particular, samples from the Octyl-Sepharose™ column were analysed on polyacrylamide gels (9%) under reducing conditions¹⁷. A mixture of molecular weight markers containing myosin (205,000), beta-galactosidase (116,000), phosphorylase b (97,000), bovine serum albumin (66,000), ovalbumin (45,000) and carbonic anhydrase (29,000) was run on each gel. After electrophoresis, gels were either stained with coomassie blue, fixed and dried or blotted onto nitrocellulose membranes.

In some experiments, protein bands of interest were cut out from the stained gel and the protein electroeluted (40V and 70mA for 20 hours) into 0.1M sodium bicarbonate pH 7.8 containing 0.1% SDS. The eluted material was then dialysed extensively against distilled water, dried under vacuum and stored at -40°C.

After proteins were blotted onto nitrocellulose membranes, the membranes were cut into strips and incubated in phosphate buffered saline (PBS) at 37°C overnight with gentle shaking. The membranes were then reacted with 10% newborn calf serum (NCS) in PBS containing 0.5% Tween™ 20 for 2h at 37°C with gentle shaking. After rinsing and washing with PBS containing 0.5% Tween™ 20 (3 x 5ml) followed by reaction with serum from a patient with high levels of adrenal autoantibodies or with normal serum diluted 1:400 in 10% NCS, 10% glycerol, 1M glucose in PBS with 0.5% Tween™ 20 for 2h at 37°C with gentle shaking, the membranes were washed extensively with PBS containing 0.5% Tween™ 20. They were then incubated with dilute anti-human immunoglobulin-horseradish peroxidase conjugate in PBS containing 0.05% Tween™ 20 for 1h at room temperature with gentle shaking. This was followed by washing with PBS containing 0.5% Tween™ 20 and reaction with a chemiluminescence generating substrate according to the substrate manufacturer's protocol (ECL System from Amersham). Nitrocellulose membrane strips and photographic film were set for 10-60 seconds and then developed.

Experiments were also performed in which rabbit antibody to recombinant steroid 21-hydroxylase⁵⁶ and rabbit antibody to microsomal epoxide hydrolase⁵⁷ were used in combination with anti-rabbit IgG horseradish peroxidase conjugate.

### Example 3

### Analysis of Electrophoresis Gel

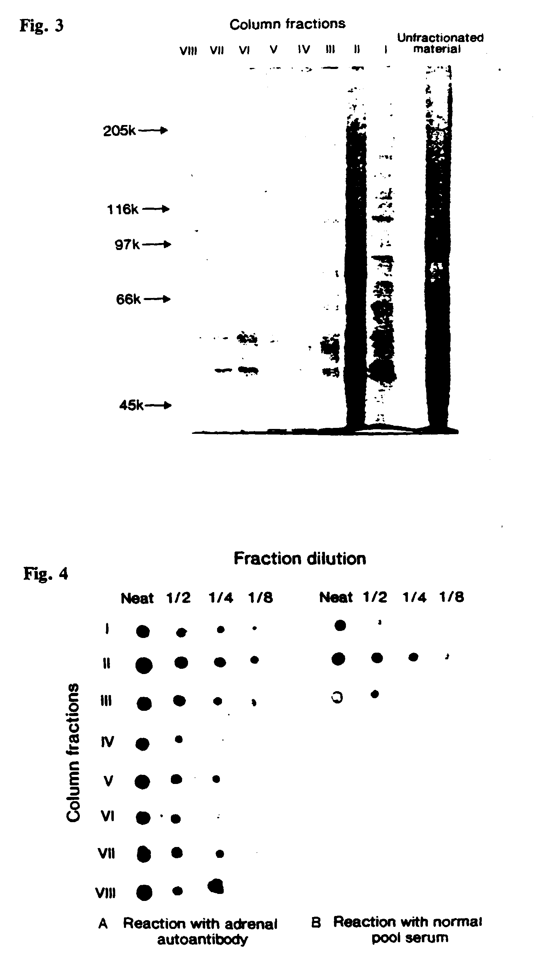

As can be seen in Fig. 2, the adrenal autoantibodies only reacted with protein bands with an observed molecular weight of 55,000 ± 5,000. In further studies, the 55,000 mol. wt. band of fraction VI was cut out of the gel, electroeluted and re-run in the gel electrophoresis system. This showed that the protein migrated as a single band with mol. wt. 55,000. In addition, when the electroeluted material was blotted into nitrocellulose, it reacted strongly with adrenal autoantibodies. These studies indicated that 55,000 molecular weight protein is the major adrenal autoantigen involved in autoimmune Addison's disease.

Protein staining of the gel obtained by electrophoresis of the column fractions showed that fraction VI contained a protein with a molecular weight of 55,000 (Fig. 3). Some of the fractions surrounding fraction VI also contained smaller amounts of the 55,000 mol. wt protein.

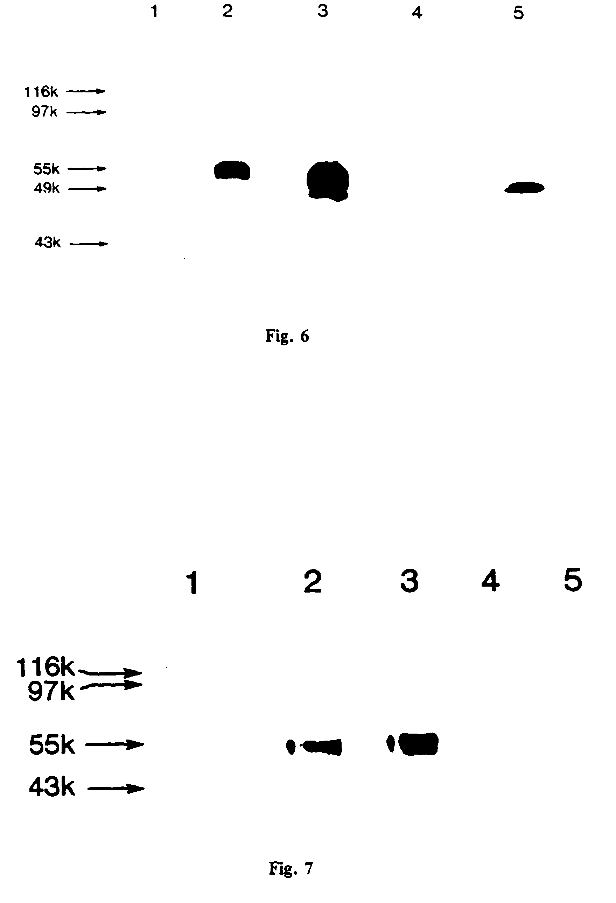

A further Western blot analysis of fraction VI is shown in Figure 6, in which Lane 1 shows reaction with normal pool serum, lane 2 reaction with adrenal autoantibody positive pool serum, lane 3 reaction with rabbit 21-hydroxylase antibody, lane 4 reaction with normal rabbit serum and lane 5 reaction with rabbit epoxide hydrolase antibody. The rabbit antibody to 21-hydroxylase, is shown to react with the 55,000 mol. wt. protein whereas the antibody to epoxide hydrolase reacted with a protein of molecular weight 49,000.

In some experiments, the 55k band from SDS-PAGE analysis of fraction VI was cut out of the gel, electroeluted, run on PAGE to confirm homogeneity (data not shown) and analysed by Western blot. The eluted band reacted strongly with adrenal autoantibody positive serum and with rabbit antibody to 21-hydroxylase but not with epoxide hydrolase antibody nor with normal pool human serum, nor with normal rabbit serum.

These observations indicate that the autoantigen is steroid 21-hydroxylase and that a previous suggestion that it is epoxide hydrolase (UK patent application No 9205040.0) resulted from sequencing the wrong band on a gel.

### Example 4

### Absorption of Adrenal Antigen with Adrenal Antibodies.

IgG was purified from normal pool sera and individual patient sera using chromatography on protein A-glass bead columns (Bioprocessing Consett, UK). Aliquots of purified adrenal protein were incubated with the IgGs (2-5mgml⁻¹) for 1h at 37°C. A protein A-glass bead suspension in PBS was then added to bind free IgG and IgG complexed to adrenal antigen(s). After 1h at room temperature, the mixture was centrifuged (12,000xg, 10mm, 4°C) and the supernatants analysed by SDS-PAGE and Western blotting using Addison sera and rabbit antibodies to recombinant 21-hydroxylase.

Analysis of the Western blots indicated that rabbit antibody to recombinant 21-hydroxylase did not react with native adrenal 55k protein which had been pre-absorbed with IgG from human adrenal autoantibody positive Addison sera (data not shown). Pre-absorption with normal pool serum IgG had no effect on reactivity with 21-hydroxylase antibody.

### Example 5

### Assay for adrenal autoantibodies using adrenal autoantigen.

Multiple small (1µl) aliquots of fractions I-VIII from the experiment shown in Fig. 1 were blotted into nitrocellulose membranes. The membranes were cut into strips and washed and reacted with serum containing adrenal autoantibodies or normal pool serum.

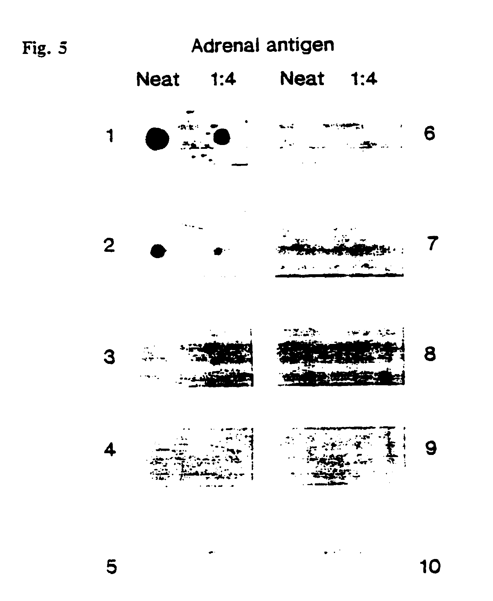

As can be seen in Fig. 4B, dot blots of fractions I-III reacted strongly with normal pool serum and there was a weak interaction between fraction IV and normal pool serum. The Western blotting studies shown in Fig. 2 indicated that this effect of normal human serum was not due to an interaction with adrenal autoantigen. However, fractions I-IV were clearly unsuitable for use in analysis of serum adrenal autoantibodies. Fraction VI however did not show any interaction with normal pool serum in the dot blot system, suggesting that it could be used in a specific assay system for adrenal autoantibodies. Typical results using fraction VI in a dot-blot assay are shown in Fig. 5. The dot-blot assay whose results are shown in Fig. 5 was conducted using ten serum samples from different patients, as follows: samples 1 & 2: Addison's disease; sample 3: normal pool; samples 4 & 5: systemic lupus erythematoses; sample 6: Hashimoto's disease; samples 7 & 8: primary biliary cirrhosis; samples 9 & 10: Graves' disease.

Table 1 summarises data obtained with a group of Addison patients. Out of the 36 Addison sera analysed by dot blot assay, 26 (72%) were positive. Analysis of the sera by one or more of three different methods (immunofluorescence, immunoprecipitation or Western blot) confirmed the presence of adrenal autoantibodies in all 26 dot blot positive sera (Table 1). The 10 Addison sera negative by dot blot assay were also negative in the three other systems used to detect adrenal autoantibodies (Table 1). The 26 adrenal autoantibody positive patients had a high proportion of thyroid autoantibodies. In particular, TPO (thyroid peroxidase) autoantibodies in 22/26 (85%), Tg (thyroglobulin) autoantibodies in 17/26 (65%) and TRAb (thyroid stimulating hormone receptor antibodies) in 4/26 (15%). At least two of the TRAb positive patients (nos. 23 and 26) had well-documentated histories of thyrotoxicosis. In contrast, the 10 Addison patients shown in Table 1 who were adrenal autoantibody negative had a similar proportion of thyroid autoantibodies as the normal population¹⁸.

In dot blot assays of sera from 10 patients with Hashimoto's disease, 10 patients with Graves' disease, 5 patients with rheumatoid arthritis, 5 patients with systemic lupus erythematoses, 4 patients with primary biliary cirrhosis and 10 normal individuals, only one serum (from a patient with Graves' disease) was found to be positive. This positive Graves' serum was confirmed to have antibodies reactive with the 55,000 band on Western blot.

Our observations with the dot blot assay are in good agreement with previous studies of large numbers of sera using immunofluorescence^{2,19-23}. For example, Nerup²⁰ and Blizzard² found that respectively 74% and 53% of patients with idiopathic Addison's disease had serum adrenal autoantibodies by immunofluorescene. Healthy normal donors rarely have adrenal autoantibodies detectable by immunofluorescence but most studies report adrenal autoantibodies in a few patients with non-adrenal autoimmune disease, including Graves' disease²⁰⁻²³. This is consistent with the detection of adrenal autoantibodies by dot blot in one Graves' serum.

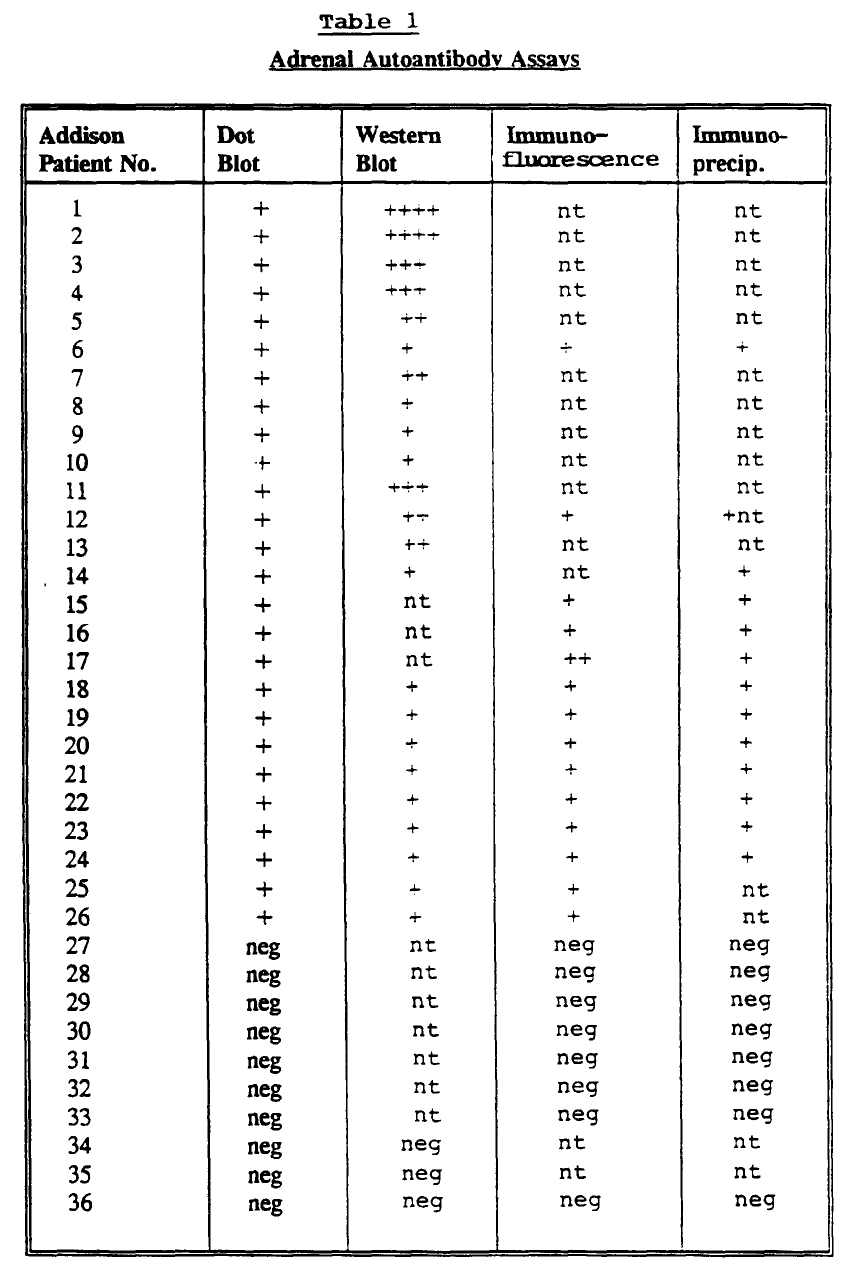

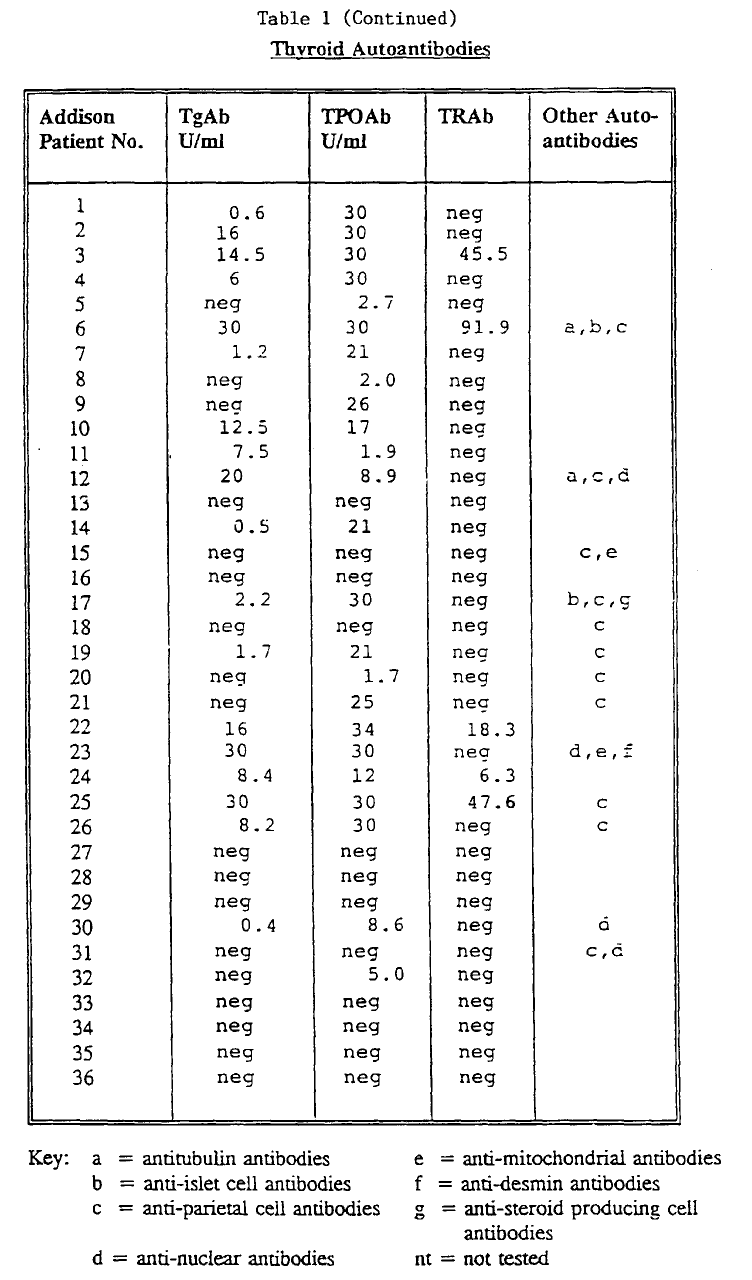

### Example 6

### Expression of 21-hydroxylase in Saccharomyces cerevisiae

For expression in the yeast Saccharomyces cerevisiae, the plasmid yeast vector pYES 2.0 (obtained from Invitrogen) was used. The steroid 21-hydroxylase gene sequence in the plasmid vector pCD//pC21/3c¹⁰ was obtained from the American Type Culture Collection, Rockville, USA (Deposit No 57421). The 21-hydroxylase gene sequence was subcloned as BamHI fragments (BamHI obtained from Northumbria Biologicals Ltd. Cramlington NE23 9HL, UK) into the cloning vector pTZ18 (obtained from Pharmacia).

After subcloning, further digestion was carried out using the enzyme SphI (Northumbria Biologicals Ltd. Cramlington NE23 9HL, UK) followed by NarI (Northumbria Biologicals Ltd, Cramlington NE23 9HL, UK) to obtain a large fragment comprising the entire coding region of the 21-hydroxylase gene apart from 41 base pairs at the 5' end.

The 21-hydroxylase gene so prepared was then ligated into the plasmid yeast vector pYES (cut with the enzymes BamHI and SphI) using a linker molecule consisting of parts of the STE2 gene (11 base pairs of non-coding sequence and 42 base pairs of coding sequence)⁵⁸. The linker molecule was synthesised using an oligonucleotide synthesiser.

The 21-hydroxylase gene incorporated into the plasmid yeast vector in this way was then incorporated into yeast cells (Saccharomyces cerevisiae C13 ABYS 8B, Yeast Genetic Stock Center, Berkeley, California) by electroporation (equipment obtained from Invitrogen) and the cells grown at 30°C for 48h on selective media (YND-glucose supplemented with amino acids except for uracil). The yeast cultures grown on selective media were used to inoculate larger expression cultures in YEP-glucose (2%) or YEP galaclose (2%).

Yeast cells from 100ml 48h expression cultures were harvested at late-log phase, washed in 50mM Tris-HC1 pH 8.0 with addition of 1mM phenyl-methylsulphonyl-fluoride (PMSF) and resuspended in 1 ml of 1 % sodium deoxychotate in 50mM Tris-HC1, pH 8.0 containing 1mM PMSF. Acid treated beads (Sigma 450-500um) were added and the cells broken by vortexing 10 times for 30sec with 30sec incubation on ice between such vortex period. After the removal of glass beads, the homogenates were centrifuged at 12.000 x g for 2min and the supernatants analysed on SDS-PAGE followed by Western blotting.

### Bibliography

1. Anderson JR, Goudie RB, Gray KG, Timbury GC (1957) Lancet i: 1123-1124
2. Blizzard RM, Kyle M (1963) J. Clin. Invest. 42:1653-1660.
3. Goudie RB, McDonald E, Anderson JR, Gray K (1968) Clin. exp. Immunol. 3:119-131.
4. Sotosiou F, Bottazzo GF, Doniach D (1980) Clin. exp. Immunol. 39:97-111.
5. Stechemesser E, Scherbaum WA, Grossmann T, Berg PA (1985) J. Immunol. Methods 80:67-76.
6. Kosowicz J, Gryczynska M, Bottazzo GF (1986) Clin. exp. Immunol. 63:671-679.
7. Centeno E R, Shulman S (1973) Immunology 24 : 901-910
8. Bright G M and Singh I (1990) J Clin. Endocrinol. Merab. 70(1) : 95-99
9. U.S. Patent No 4720454, White PC et al.
10. White PC, New MI, Dupont B (1986) Proc. Natl. Acad. Sci. USA 83 : 5111-5115
11. Furmaniak J, Talbot D, Reinwein D, Benker G, Creagh FM, Rees Smith B (1988) FEBS Letts 231:25-28.
12. Schardt CW, McLachlan SM, Matherson J, Rees Smith B (1982) J Immunol, Methods 55:155-168.
13. Kajita Y, Morgan D, Parkes AB, Rees Smith B (1985) FEBS Letts 187:334-338.
14. Bradford M (1976) Anal. Biochem. 72:248-254.
15. Kominami S, Ocho H, Kobayashi Y. Takemori S (1980) J Biol Chem. 255(8):3386-3394.
16. Bumpus JA, Dus KM (1982) J. Biol. Chem. 257(21):12696-12704.
17. Laemmli UK (1970) Nature 227:680-684.
18. Prentice LM, Phillips DIW, Sarsero D, Beever K, McLachlan SM, Rees Smith B (1990) Acta Endocrinol. 123:493-498.
19. Blizzard RM, Chee D, Davis W (1966) Clin. exp. Immunol. 1:119-128.
20. Nerup J (1974) Ada Endocrinol. (1974) 76: 142-158.
21. Scherbaum WA, Berg PA (1982) Clin. Endocrinol. 16:345-352.
22. Betterle C, Zamchetta R, Trevisan A, Zanette F, Pedini B, Mantero F (4th June 1983) Lancet: 1238-1240.
23. Betterle C, Scalici C, Presotto, F, Pedini B, Moro L, Rigouo F, Mantero F (1988) J. Endocrinol 117:467-475.
24. Lather R F, Lecoq J P in Genetric Engineering, Academic Press 1983 (pp31-50).
25. Smith M, Gillam S in Genetic Engineering : Principles and Methods, Plenum Press (1981) 3:1-32.
26. Dalbadie-McFarland et al. (1982), Proc. Natl. Acad. Sci. USA 79:6409-13.
27. Gillam et al. (1980) Gene 12:129-137.
28. Zoller M J, Smith M (1983) Meth. Enzymol. 100:468-500.
29. Kohler G, Milstein C (1975) Nature 256:495-497.
30. Kohler G, Milstein C (1976) Eur. J. Immunol. 6:511-519.
31. Kohler G, Milstein C (1976) Eur. J Immunol. 6:292.
32. Hammerling et at in Monoclonal Antibodies and T-Cell Hybridomas, Elsevier, New York 1981 (pp 563-681).
33. Kennett R H, McKearn T J, Bechtol K B (editors), Monoclonal Antibodies, Plenum Press, New York (1980).
34. Melchers F, Potter M, Warner N (editors), Lymphocyte Hybridomas, Curr. Top. Microbiol. and Immunol, Springer-Verlag, Berlin 81:1-246(1978).
35. Vunakis H V, Langone J L (editors), Methods in Enzmology, Academic Press, Vols. 70, 73, 74, 84 and 92 [Immunochemical Techniques parts A (1980), B (1981), C (1981), D (1982), E (1983)].
36. Janeway C (1989) Nature 341:482.
37. Acha-Orbea H et al. (1989) Ann. Rev. Immunol 7:371-405.
38. Kumar V et al. (1989) Ann. Rev. Immunol 7:657-682.
39. Urban J L et al. (1989) Cell 54:577-592.
40. Wraith D C et al. (1989) Cell 57:709-715.
41. Wraith D C et al. (1989) Cell 59:247-255.
42. Urban J L et al. (1989) Cell 59:257-271.
43. Cohen I R (1986) Immunol. Rev. 94:5-21.
44. (1986) Prog. Immunol. VI:491-499.
45. (1988) Scientific Amer. 258:52-60.
46. (Feb 15, 1989) Hosp. Prac. 57-64.
47. Cohen I R et al. (1988) Immunol. Today 9:332-335.
48. Vandenbark AA et al. (1989) Nature 341:541-544.
49. Howell M D et al. (1989) Science 246:668-671.
50. Sun D et al. (1988) Nature 332:843-845.
51. Sun D et al. (1988) Eur. J. Immunol. 18:1993-1999.
52. Green D et al. (1983) Ann. Rev. Immunol. 1:439.
53. Benacerraf B in The Biology of Immunologic Disease, H P Publishing Co Inc, New York 1983 (49-62).
54. Burns F J et al. (1989) J. Exp. Med. 169:27.
55. Acha-Orbea H et al. (1989) Ann. Rev. Immunol. 7:371.
56. Hu M, Chung B (1990) Mol. Endocrinol 4 : 893-898
57. Craft J A, Jackson M R, Burchell B (1987) Biochem. Soc. Trans. 15 : 708-709
58. King K, Dohlman H G, Thorner J, Caron M G, Lefkowitz R J (1990) Science 250 : 121-123
59. J. Fambrook; E F Fritch and T Maniatis, Molecular Cloning - A Laboratory Manual. Cold Spring Harbor Laboratory, Cold Spring Harbor, New York, Second Edition 1989.

## Claims

1. A method for detecting adrenal autoantibody in a sample, comprising contacting with the sample:
(a) a protein comprising an epitope for adrenal autoantibody, having an observed molecular weight of from about 50,000 to about 60,000 and obtainable by:
homogenising mammalian adrenal glands,
subjecting the homogenate to differential centrifugation to obtain a microsome fraction,
suspending the microsome fraction in a phosphate buffer,
centrifuging the suspension in the presence of sodium cholate to form a supernatant,
adding polyethylene glycol and further sodium cholate to the supernatant and mixing the supernatant,
centrifuging the thus mixed supernatant to form a pellet,
resuspending the pellet in aqueous sodium cholate to form a suspension,
dialysing the suspension against aqueous sodium cholate to form a solubilised microsome preparation, and
purifying the solubilised microsome preparation by chromatography on an Octyl-Sepharose™ CL-4B column equilibrated with 50 mM phosphate buffer, pH 7.0, 20% glycerol, 0.1 mM EDTA, 0.1 mM DTT, and eluted with 0.2% Emulgen™ to obtain a column fraction corresponding to peak VI of Figure 1;
(b) the adrenal protein (a) modified by one or more amino acid modifications and comprising an epitope for the adrenal autoantibody; or
(c) a fragment of modified or unmodified adrenal protein (a) comprising an epitope for the adrenal autoantibody.

2. A method as claimed in claim 1, wherein the protein
(i) has a molecular weight of about 55,000, and/or
(ii) is of recombinant origin.

3. A method as claimed in claim 1, wherein there is contacted with the sample a fragment of a protein as defined in claim 2 (i) and/or (ii).

4. A method as claimed in any of claims 1 to 3, wherein the protein or protein fragment is labelled and the method further comprises detecting for labelled protein (fragment)/autoantibody complex or unbound labelled protein (fragment).

5. A method as claimed in any of claims 1 to 3 and which further comprises contacting the protein or protein fragment and the sample with labelled antibody to the protein or protein fragment and then detecting labelled antibody bound to the protein or protein fragment, the protein or protein fragment optionally being immobilised on a substrate.

6. A method as claimed in any of claims 1 to 3, wherein the protein or protein fragment is immobilised on a substrate and the method further comprises, after contacting the protein or protein fragment with labelled ligand for the adrenal autoantibody and detecting bound labelled ligand, the ligand optionally being a protein or protein fragment as defined in claim 1, an antibody to the adrenal autoantibody, an anti-immunoglobulin, protein A or protein G.

7. A method as claimed in any one of claims 1 to 6, wherein the protein or protein fragment is human.

8. A method for detecting adrenal autoantibodies in a sample, comprising:
contacting with the sample immobilised antibody against a protein as defined in any of claims 1, 2 or 7 or a protein fragment as defined in any of claims 1 to 3 or 7, which antibody has bound thereto a labelled ligand comprising a protein as defined in any of claims 1, 2 or 7 or a protein fragment as defined in any of claims 1 to 3 or 7, and detecting labelled ligand bound to immobilised antibody or labelled ligand bound to antibody in the sample.

9. A protein fragment as defined in any of claims 1(c) or claim 3 and which is labelled.

10. A synthetic peptide comprising an epitope to adrenal autoantibody and obtainable from a protein as defined in claim 1(a).

11. A kit for detecting adrenal autoantibody in a sample, comprising:
(i) a protein as defined in any of claims 1, 2 or 7 or a protein fragment as defined in any of claims 1 to 3 or 7; or
(ii) immobilised antibody against a protein as defined in any of claims 1, 2 or 7 or against a protein fragment as defined in any of claims 1 to 3 or 7 having bound thereto a labelled ligand comprising a protein as defined in any of claims 1, 2 or 7 or a protein fragment as defined in any of claims 1 to 3 or 7.

12. A kit as claimed in claim 11(i), wherein:
(i) the protein or protein fragment is labelled; or
(ii) the kit further comprises a labelled antibody to the protein or protein fragment and wherein the protein or protein fragment is optionally immobilised on a substrate; or
(iii) the protein or protein fragment is immobilised on a substrate and the kit further comprises a labelled ligand for the adrenal autoantibody, the ligand optionally being a protein or protein fragment as defined in claim 1, an antibody to the adrenal autoantibody, an anti-immunoglobulin, protein A or protein G.

13. A pharmaceutical composition comprising as active ingredient a protein as defined in any of claims 1, 2 or 7 or a protein fragment as defined in any of claims 1 to 3 or 7 and a pharmaceutically acceptable diluent, excipient or carrier.

14. A pharmaceutical composition comprising a ligand which is a protein or protein fragment containing a T cell epitope of the autoantigen defined in claim 1(a) and capable of binding to the receptor of an adrenal autoantigen specific T cell, and a pharmaceutically acceptable diluent, excipient or carrier.

15. An isolated T cell specific for a protein as defined in claim 1(a) obtainable from the microsome fraction of human or non-human mammal adrenal glands and having an observed molecular weight of about 50,000 to 60,000 and antigenic towards adrenal autoantibody.

16. A protein or protein fragment which mimicks the receptor of a T cell specific for a protein as defined in claim 1(a) obtainable from the microsome fraction of human or non-human mammal adrenal glands and having an observed molecular weight of about 50,000 to 60,000 and antigenic towards adrenal autoantibody.

17. An isolated T cell induced by a T cell as claimed in claim 15 or by a protein or protein fragment as claimed in claim 16.

18. An isolated Ts cell specific for a B cell or T cell specific for a protein as defined in claim 1(a) obtainable from the microsome fraction of human or non-human mammal adrenal glands and having an observed molecular weight of about 50,000 to 60,000 and antigenic towards adrenal autoantibody.

19. A pharmaceutical composition comprising T cells specific for a protein as defined in claim 1(a), T cells induced by T cells specific for a protein as defined in claim 1(a), Ts cells specific for B cells or T cells specific in either case for a protein as defined in claim 1(a), or a protein or protein fragment which mimicks the receptor of such a T cell and a pharmaceutically acceptable diluent, excipient or carrier.

## Patentansprüche

1. Verfahren zum Nachweis von adrenalem Autoantikörper in einer Probe, umfassend Kontaktieren der Probe mit:
(a) einem Protein, umfassend ein Epitop für adrenalen Autoantikörper mit einem gemessenen Molekulargewicht von etwa 50.000 bis etwa 60.000 und erhältlich durch:
Homogenisieren von Säuger-Nebennieren,
Differentialzentrifugation des Homogenats, um eine Mikrosomenfraktion zu erhalten,
Suspendieren der Mikrosomenfraktion in einem Phosphatpuffer,
Zentrifugation der Suspension in Gegenwart von Natriumcholat für die Bildung eines Überstands,
Zugabe von Polyethylenglykol und weiterem Natriumcholat zu dem Überstand und Mischen des Überstands,
Zentrifugation des gemischten Überstands zur Bildung eines Niederschlags,
Suspendieren des Niederschlags in wässrigem Natriumcholat zur Bildung einer Suspension,
Dialyse der Suspension gegen wässriges Natriumcholat zur Bildung einer solubilisierten Mikrosomenpräparation, und
Reinigung der solubilisierten Mikrosomenpräparation durch Chromatographie auf einer Octyl-Sepharose™ CL-4B-Säule, die mit 50 mM Phosphatpuffer, pH 7,0, 20% Glycerin, 0,1 mM EDTA, 0,1 mM DTT equilibriert und mit 0,2% Emulgen™ eluiert wird, um eine dem Ausschlag VI von Figur 1 entsprechende Säulenfraktion zu erhalten;
(b) dem adrenalen Protein (a), das durch eine oder mehrere Aminosäuremodifikationen modifiziert ist und ein Epitop für den adrenalen Autoantikörper umfasst; oder
(c) einem Fragment des modifizierten oder nicht-modifizierten adrenalen Proteins (a), umfassend ein Epitop für den adrenalen Autoantikörper.

2. Verfahren gemäß Anspruch 1, wobei das Protein
(i) ein Molekulargewicht von etwa 55.000 hat, und/oder
(ii) rekombinanten Ursprungs ist.

3. Verfahren gemäß Anspruch 1, wobei ein Fragment eines in Anspruch 2 (i) und/oder (ii) definierten Proteins mit der Probe in Kontakt gebracht wird.

4. Verfahren gemäß einem der Ansprüche 1 bis 3, wobei das Protein oder Proteinfragment markiert ist und das Verfahren weiter den Nachweis von markiertem Protein(fragment)/Autoantikörper-Komplex oder nicht-gebundenem, markiertem Protein(fragment) umfasst.

5. Verfahren gemäß einem der Ansprüche 1 bis 3, weiter umfassend die Kontaktierung des Proteins oder Proteinfragments und der Probe mit markiertem Antikörper gegen das Protein oder Proteinfragment und einen darauffolgenden Nachweis von markiertem, an das Protein oder Proteinfragment gebundenem Antikörper, wobei das Protein oder Proteinfragment gegebenenfalls auf einem Substrat immobilisiert vorliegt.

6. Verfahren gemäß einem der Ansprüche 1 bis 3, wobei das Protein oder Proteinfragment auf einem Substrat immobilisiert ist und das Verfahren weiter den Nachweis von gebundenem, markiertem Ligand nach Kontaktieren des Proteins oder Proteinfragments mit markiertem Ligand für den adrenalen Autoantikörper umfasst, wobei der Ligand gegebenenfalls ein in Anspruch 1 definiertes Protein oder Proteinfragment, ein Antikörper gegen den adrenalen Autoantikörper, ein Anti-Immunglobulin, Protein A oder Protein G ist.

7. Verfahren gemäß einem der Ansprüche 1 bis 6, wobei das Protein oder Proteinfragment menschlichen Ursprungs ist.

8. Verfahren zum Nachweis von adrenalen Autoantikörpern in einer Probe, umfassend:
Kontaktieren von immobilisiertem Antikörper gegen ein in einem der Ansprüche 1, 2 oder 7 definiertes Protein oder ein in einem der Ansprüche 1 bis 3 oder 7 definiertes Proteinfragment mit der Probe, wobei der Antikörper einen markierten Liganden gebunden hat, umfassend ein in einem der Ansprüche 1, 2 oder 7 definiertes Protein oder ein in einem der Ansprüche 1 bis 3 oder 7 definiertes Proteinfragment und Nachweis von markiertem Ligand, der an den immobilisierten Antikörper gebunden ist oder von markiertem Ligand, der an Antikörper in der Probe gebunden ist.

9. Proteinfragment, definiert in einem der Ansprüche 1(c) oder 3, das markiert ist.

10. Synthetisches Peptid, umfassend ein Epitop für adrenalen Autoantikörper und erhältlich von einem in Anspruch 1(a) definierten Protein.

11. Kit zum Nachweis von adrenalem Autoantikörper in einer Probe, umfassend:
(i) ein in einem der Ansprüche 1, 2 oder 7 definiertes Protein oder ein in einem der Ansprüche 1 bis 3 oder 7 definiertes Proteinfragment, oder
(ii) immobilisierten Antikörper gegen ein in einem der Ansprüche 1, 2 oder 7 definiertes Protein oder gegen ein in einem der Ansprüche 1 bis 3 oder 7 definiertes Proteinfragment mit einem gebundenen, markierten Ligand, umfassend ein in einem der Ansprüche 1, 2 oder 7 definiertes Protein oder ein in einem der Ansprüche 1 bis 3 oder 7 definiertes Proteinfragment.

12. Kit gemäß Anspruch 11(i), wobei
(i) das Protein oder Proteinfragment markiert ist oder
(ii) der Kit ferner einen markierten Antikörper gegen das Protein oder Proteinfragment umfasst, wobei das Protein oder Proteinfragment gegebenenfalls auf einem Substrat immobilisiert ist, oder
(iii) das Protein oder Proteinfragment auf einem Substrat immobilisiert ist und der Kit ferner einen markierten Liganden für den adrenalen Autoantikörper, wobei der Ligand gegebenenfalls ein in Anspruch 1 definiertes Protein oder Proteinfragment ist, einen Antikörper gegen den adrenalen Autoantikörper, ein Anti-Immunglobulin, Protein A oder Protein G umfasst.

13. Pharmazeutische Zusammensetzung, umfassend als aktiven Bestandteil ein in einem der Ansprüche 1, 2 oder 7 definiertes Protein oder ein in einem der Ansprüche 1 bis 3 oder 7 definiertes Proteinfragment und ein pharmazeutisch verträgliches Verdünnungsmittel, Exzipiens oder Träger.

14. Pharmazeutische Zusammensetzung, umfassend einen Ligand, der ein Protein oder Proteinfragment mit einem T-Zell-Epitop des in Anspruch 1(a) definierten Autoantigens ist und zur Bindung an den Rezeptor einer adrenalen Autoantigen-spezifischen T-Zelle fähig ist, und ein pharmazeutisch verträgliches Verdünnungsmittel, Exzipiens oder Träger.

15. Isolierte T-Zelle, die für ein in Anspruch 1(a) definiertes Protein spezifisch ist, das aus der Mikrosomenfraktion von menschlichen oder nicht-menschlichen Säuger-Nebennieren erhältlich ist, ein gemessenes Molekulargewicht von etwa 50.000 bis 60.000 aufweist und antigen gegenüber adrenalem Autoantikörper ist.

16. Protein oder Proteinfragment, das den Rezeptor einer T-Zelle nachahmt, die spezifisch für ein in Anspruch 1(a) definiertes Protein ist, das aus der Mikrosomenfraktion von menschlichen oder nicht-menschlichen Säuger-Nebennieren erhältlich ist, ein gemessenes Molekulargewicht von etwa 50.000 bis 60.000 aufweist und antigen gegenüber adrenalem Autoantikörper ist.

17. Isolierte T-Zelle, induziert durch eine T-Zelle gemäß Anspruch 15 oder durch ein Protein oder Proteinfragment gemäß Anspruch 16.

18. Isolierte Ts-Zelle, spezifisch für eine B-Zelle oder T-Zelle, die spezifisch für ein in Anspruch 1(a) definiertes Protein ist, das aus der Mikrosomenfraktion von menschlichen oder nicht-menschlichen Säuger-Nebennieren erhältlich ist, ein gemessenes Molekulargewicht von etwa 50.000 bis 60.000 aufweist und antigen gegenüber adrenalem Autoantikörper ist.

19. Pharmazeutische Zusammensetzung, umfassend für ein in Anspruch 1(a) definiertes Protein spezifische T-Zellen, durch für ein in Anspruch 1(a) definiertes Protein spezifische T-Zellen induzierte T-Zellen, Ts-Zellen, spezifisch für B-Zellen oder T-Zellen, die für ein in Anspruch 1(a) definiertes Protein oder ein Protein oder Proteinfragment, das den Rezeptor einer derartigen T-Zelle nachahmt, spezifisch sind, und ein pharmazeutisch verträgliches Verdünnungamittel, Exzipiens oder Träger.

## Revendications

1. Procédé de détection d'un auto-anticorps surrénal dans un échantillon, selon lequel on met en contact avec l'échantillon :
(a) une protéine comprenant un épitope pour l'auto-anticorps surrénal, ayant un poids moléculaire observé d'environ 50 000 à environ 60 000, et pouvant être obtenue par :
homogénéisation des glandes surrénales de mammifère, en soumettant l'homogénat à une centrifugation différentielle pour obtenir une fraction à base de microsomes,
mise en suspension la fraction à base de microsomes dans un tampon de type phosphate,
centrifugation de la suspension en présence de chlorate de sodium pour former un surnageant,
ajout de polyéthylène glycol, et ensuite de cholate de sodium dans le surnageant, et mélange du surnageant,
centrifugation du surnageant ainsi mélangé pour former une pastille,
remise en suspension de la pastille dans du cholate de sodium aqueux pour former une suspension,
dialyse de la suspension contre du cholate de sodium aqueux afin de former un produit à base de microsomes dissous, et
purification du produit à base de microsomes dissous par chromatographie sur une colonne d'Octyl-Sepharose (marque commerciale) CL-4B avec un tampon de type phosphate 50 mM, à un pH de 7,0 avec 20 % de glycérol, de l'EDTA 0,1 mM et du DTT 0,1 mM, et en éluant avec 0,2 % d'Emulgen (marque commerciale) pour obtenir une fraction de colonne correspondant au pic VI sur la figure 1 ;
(b) la protéine surrénale (a) modifiée par une ou plusieurs modifications d'aminoacide, et comprenant un épitope pour l'auto-anticorps surrénal ; ou
(c) un fragment de la protéine surrénale (a) modifiée ou non modifiée, comprenant un épitope pour l'auto-anticorps surrénal.

2. Procédé selon la revendication 1, dans lequel la protéine :
(i) a un poids moléculaire d'environ 55 000, et/ou
(ii) est dérivée d'un recombinant.

3. Procédé selon la revendication 1, dans lequel on met en contact avec l'échantillon, un fragment d'une protéine tel que défini dans la revendication 2, (i) et/ou (ii).

4. Procédé selon l'une quelconque des revendications 1 à 3, dans lequel la protéine ou le fragment de protéine est marqué, et où le procédé comprend en outre la détection d'un complexe de protéine marquée (fragment) et d'auto-anticorps ou d'une protéine marquée non liée (fragment).

5. Procédé selon l'une quelconque des revendications 1 à 3, et comprenant en outre une mise en contact de la protéine ou du fragment de protéine et de l'échantillon, avec un anticorps marqué dirigé contre la protéine ou le fragment de protéine, et où on détecte ensuite l'anticorps marqué lié à la protéine ou au fragment de protéine, la protéine ou le fragment de protéine étant éventuellement immobilisé(e) sur un substrat.

6. Procédé selon l'une quelconque des revendications 1 à 3, dans lequel la protéine ou le fragment de protéine est immobilisé sur un substrat, et où le procédé comprend en outre une mise en contact de la protéine ou du fragment de protéine avec un ligand marqué pour l'auto-anticorps surrénal, et une détection du ligand marqué et lié, le ligand étant éventuellement une protéine ou un fragment de protéine tel que défini dans la revendication 1, un anticorps dirigé contre l'auto-anticorps surrénal, une anti-immunoglobuline, la protéine A ou la protéine G.

7. Procédé selon l'une quelconque des revendications 1 à 6, dans lequel la protéine ou le fragment de protéine est humain.

8. Procédé de détection d'auto-anticorps surrénaux dans un échantillon, comprenant :
une mise en contact avec l'échantillon, d'un anticorps immobilisé dirigé contre une protéine selon l'une quelconque des revendications 1, 2 ou 7, ou un fragment de protéine tel que défini dans l'une quelconque des revendications 1 à 3 ou 7, cet anticorps étant lié à un ligand marqué comprenant une protéine selon l'une quelconque des revendications 1, 2 ou 7, ou un fragment de protéine selon l'une quelconque des revendications 1 à 3 ou 7, et une détection du ligand marqué lié à l'anticorps immobilisé ou du ligand marqué lié à un anticorps dans l'échantillon.

9. Fragment de protéine selon la revendication 1(c) ou la revendication 3, et qui est marqué.

10. Peptide synthétique comprenant un épitope pour un auto-anticorps surrénal, pouvant être obtenu à partir d'une protéine telle que définie dans la revendication 1(a).

11. Nécessaire pour détecter un auto-anticorps surrénal dans un échantillon, comprenant :
(i) une protéine telle que définie dans la revendication 1, 2 ou 7, ou un fragment de protéine tel que défini dans l'une quelconque des revendications 1 à 3 ou 7 ; ou
(ii) un anticorps immobilisé dirigé contre une protéine telle que définie dans la revendication 1, 2 ou 7, ou contre un fragment de protéine tel que défini dans l'une quelconque des revendications 1 à 3 ou 7 auquel est lié un ligand marqué comprenant une protéine telle que définie dans la revendication 1, 2 ou 7, ou un fragment de protéine tel que défini dans l'une quelconque des revendications 1 à 3 ou 7.

12. Nécessaire selon la revendication 11(i), dans lequel :
(i) la protéine ou le fragment de protéine est marqué ; ou
(ii) le nécessaire comprend en outre un anticorps marqué dirigé contre la protéine ou le fragment de protéine, et dans lequel la protéine ou le fragment de protéine est éventuellement immobilisé sur un substrat ; ou
(iii) la protéine ou le fragment de protéine est immobilisé sur un substrat, et le nécessaire comprend en outre un ligand marqué pour l'auto-anticorps surrénal, le ligand étant éventuellement une protéine ou un fragment de protéine tel que défini dans la revendication 1, un anticorps dirigé contre l'auto-anticorps surrénal, une anti-immunoglobuline, la protéine A ou la protéine G.

13. Composition charmaceutique comprenant comme ingrédient actif, une protéine telle que définie dans la revendication 1, 2 ou 7, ou un fragment de protéine tel que défini dans l'une quelconque des revendications 1 à 3 ou 7, et un diluant, un excipient ou un véhicule pharmaceutiquement acceptable.

14. Composition pharmaceutique comprenant un ligand qui est une protéine ou un fragment de protéine contenant un épitope pour lymphocyte T de l'auto-antigène défini dans la revendication 1(a), et capable de se lier au récepteur d'un lymphocyte T spécifique d'un auto-antigène surrénal, et un diluant, un excipient ou un véhicule pharmaceutiquement acceptable.

15. Lymphocyte T isolé spécifique d'une protéine telle que définie dans la revendication 1(a), pouvant être obtenu à partir de la fraction à base de microsomes des glandes surrénales de mammifère humain ou non humain, et ayant un poids moléculaire observé d'environ 50 000 à 60 000, et étant antigénique à l'égard d'un auto-anticorps surrénal.

16. Protéine ou fragment de protéine qui imite le récepteur d'un lymphocyte T spécifique d'une protéine telle que définie dans la revendication 1(a), qui peut être obtenu à partir de la fraction à base de microsomes des glandes surrénales de mammifère humain ou non humain et ayant un poids moléculaire observé d'environ 50 000 à 60 000, et qui est antigénique à l'égard d'un auto-anticorps surrénal.

17. Lymphocyte T isolé induit par un lymphocyte T selon la revendication 15, ou par une protéine ou un fragment de protéine selon la revendication 16.

18. Lymphocyte Ts isolé spécifique d'un lymphocyte B ou d'un lymphocyte T spécifique d'une protéine telle que définie dans la revendication 1(a), qui peut être obtenu à partir de la fraction à base de microsomes des glandes surrénales de mammifère humain ou non humain, et ayant un poids moléculaire observé d'environ 50 000 à 60 000, et étant antigénique à l'égard d'un auto-anticorps surrénal.

19. Composition pharmaceutique comprenant des lymphocytes T spécifiques d'une protéine telle que définie dans la revendication 1(a), des lymphocytes T induits par des lymphocytes T spécifiques d'une protéine telle que définie dans la revendication 1(a), des lymphocytes Ts spécifiques de lymphocytes B ou des lymphocytes T spécifiques dans chaque cas d'une protéine celle que définie dans la revendication 1(a), ou d'une protéine ou d'un fragment de protéine qui imite le récepteur d'un tel lymphocyte T, et un diluant, un excipient ou un véhicule pharmaceutiquement acceptable.
